# EUROPEAN PATENT APPLICATION

(11) **EP 4 810 024 A2**
(43) Date of publication of application: **23.09.2026**
(21) Application number: 26189565.0
(22) Date of filing: 18.04.2019
(51) Int. Cl.: A61K 9/16

(54) **DIETARY SUPPLEMENT, IN THE FORM OF A SINGLE-DOSE ORAL SOLUTION, COMPRISING MAGNESIUM, MELATONIN, TRYPTOPHAN AND 5-HYDROXYTRYPTOPHAN, USEFUL TO REDUCE THE TIME UNTIL ONSET OF SLEEP**

(30) Priority: 23.04.2018 GR 20180100167
(62) Document number(s) of the earlier application(s) in accordance with Art. 76 EPC: 19170147.3 / 3 560 353
(71) Applicant: Ioulia & Irene Tseti Pharmaceutical Laboratories Industrial and Commercial S.A., 14564 Kifissia (GR)
(72) Inventor: Tseti, Ioulia, 145 61 Kifissia (GR)
(74) Representative: Sonnenhauser, Thomas Martin

(57) **Abstract**

Dietary supplement comprising melatonin, tryptophan, 5-hydroxytryptophan and magnesium pidolate as a single-dose oral solution for direct consumption, useful for reducing the time until onset of sleep, improving biological de-synchronization and reducing fatigue. The nutritional supplement is characterized in that melatonin, tryptophan and 5-hydroxytryptophan are kept in solid form in the closure storage cap of a container based on the "closure storage cap - vial" system, while magnesium pidolate is kept in the vial of said container in liquid form. The closure storage cap is hermetically sealed onto the mouth of the vial through a thread, furthermore, the closure storage cap includes a suitable breaking mechanism, which allows the complete mixing of the solid preparation of melatonin, tryptophan and 5-hydroxytryptophan with the solution of magnesium pidolate just prior to use.

## Description

The present invention relates to a dietary supplement comprising magnesium in the form of magnesium pidolate, melatonin, tryptophan and 5-hydroxytryptophan, which are stored in a "vial - closure storage cap" system device, characterized in that the vial of the system device contains a liquid preparation comprising magnesium pidolate, a solvent, flavorings and sweeteners, preservatives and pH adjuster, while, the closure storage cap contains melatonin, tryptophan and 5-hydroxytryptophan in solid form. The closure storage cap with the active ingredients in solid form is sealed air tight apart from the solution contained in the vial and includes a suitable mechanism which allows the active substances in solid form to be mixed with the solution just prior to use. The final preparation serves the normal function of the central nervous system, reduces the time until onset to sleep and improves the effects of biological de-synchronization.

Magnesium pidolate is the commercial name of bis-[(2S)-5-oxopyrrolidine-2-carboxyl] magnesium (II) and is a magnesium (II) salt with two molecules of 2-pyrrolidone-5-carboxylic acid. From a chemical point of view, magnesium pidolate is freely soluble in water and therefore, it can be easily implemented in the preparation of pharmaceutical compositions and liquid dietary supplements. Despite the low percentage of divalent magnesium per molecule of the salt (8.66% w/w), magnesium pidolate is an excellent source of magnesium for the body because the pidolate ions can easily penetrate the cell membrane, thus achieving an effective transfer of magnesium divalent ion directly to the inner layers of the mitochondria and in the cell nucleus. Several studies show that magnesium suppresses the nervous and neuromuscular brain excitability by inhibiting N-methyl-D-aspartate (NMDA) receptors and gamma-aminobutyric acid (GABA) neurotransmitters, thus providing a beneficial effect on the management of headaches and migraines, also improving sleep time. Furthermore, magnesium regulates melatonin, a hormone, which controls the sleep-wake cycles in the body.

Melatonin, or according to IUPAC, *N*-[2-(5-methoxy-1*H*-indol-3-yl)ethyl]acetamide, is a hormone released by the pineal gland, an endocrine gland in the brain, by the amino acid tryptophan. The synthesis of melatonin in the brain occurs in a periodic manner following a daily cycle that is mainly related to the existence or absence of light. The production of melatonin reaches a maximum during the night, while, it is minimized during the day or in the presence of bright blue light. Melatonin plays a critical role in the regulation of sleep cycle by causing drowsiness and lowering body temperature. Several studies have shown that melatonin administration (<10 mg), one hour before bedtime can be effective in treating insomnia and improving sleep quality. Furthermore, melatonin reduces jet lag, namely, the disruption of the biological cycle which happens to travelers crossing multiple time zones. Recent studies revealed the antioxidant activity of melatonin acting as a strong free radical scavenger, while its interaction with the immune system is also reported. Tryptophan's adequacy in the human body plays a critical role in the biosynthesis of melatonin, as it represents its main precursor.

Tryptophan is one of the 8 "essential" amino acids for human being, as a basic building block in protein biosynthesis and plays a critical role in the development and physiological functioning of many organs in the human body. Tryptophan cannot be synthesized by human, therefore, it is necessary to be obtained through its diet. As soon as it is taken up and absorbed into the body, tryptophan is converted into its 5-hydroxy derivative, which through an enzymatic decarboxylation reaction is converted to serotonin, which in turn is metabolized to melatonin (also known as sleep hormone). Serotonin is a neurotransmitter in the brain which is related to mental functioning by contributing to feelings of happiness, while, it also relates to sleep regulation. Tryptophan's degradation leads to nicotinic acid (also known as niasin or vitamin B3) which plays a critical role in energy metabolism in the human body thus reducing the tiredness feeling. Tryptophan cannot be stored for a long time due to its direct metabolism in the human body, and for that reason, it presents one of the smallest concentrations among the necessary amino acids.

Taking into account all above, it is obvious that magnesium, melatonin and tryptophan play an important role to the normal functioning of the nervous system. Therefore, it is deemed essential to strengthen the human body with all these components. In reference to the state of the art, there is an infinite number of compositions comprising the above mentioned ingredients either individually or in combination, in the form of pharmaceutical preparations and/or food supplements. However, there is not a single reference in the state of the art claiming the use of a closure storage cap, suitable for the air tight storage of a solid mixture comprising melatonin, tryptophan and 5-hydroxytryptophan, which, said cap is hermetically sealed in the aid of a thread onto the mouth of a vial containing a magnesium pidolate oral solution, furthermore, the closure storage cap includes a suitable breaking mechanism, which allows the complete mixing of the solid preparation of melatonin, tryptophan and 5-hydroxytryptophan with the solution of magnesium pidolate to result to a single-dose oral solution, useful for reducing the time until onset of sleep, improving biological de-synchronization and reducing fatigue.

The present invention proposes a novel direct and safe administration route of a dietary supplement in the form of a single-dose oral solution, which is prepared just prior to its use by mixing a solid composition comprising melatonin, tryptophan and 5-hydroxytryptophan and a liquid preparation comprising magnesium pidolate, all enclosed in a suitable container based on the system "closure storage cap - vial". This administration route overcomes the problems encountered to people with difficulty in swallowing solid formulations such as oral granules, tablets or capsules. In addition, the present invention provides a useful device which allows the immediate delivery of the final composition therefrom.

The subject of the present invention relates to a dietary supplement comprising melatonin, tryptophan, 5-hydroxytryptophan and magnesium pidolate as a single-dose oral solution, useful for reducing the time until onset of sleep, improving biological de-synchronization and reducing fatigue. The nutritional supplement of the present invention is characterized in that it is prepared just prior to its use by dissolving a solid mixture comprising melatonin, tryptophan and 5-hydroxytryptophan in a solution of magnesium pidolate. The solid mixture comprising melatonin, tryptophan and 5-hydroxytryptophan and the solution of magnesium pidolate are stored separately up to the moment of mixing in a suitable container based on the system "closure storage cap - vial", the said storage cap contains the solid mixture of melatonin, tryptophan and 5-hydroxytryptophan and the vial contains the solution of magnesium pidolate. The storage cap is hermetically sealed in the aid of a thread onto the mouth of the vial, and it further includes a suitable breaking mechanism, which allows the complete mixing of the solid preparation of melatonin, tryptophan and 5-hydroxytryptophan with the solution of magnesium pidolate in order to form a single-dose oral solution suitable for immediate delivery from the vial.

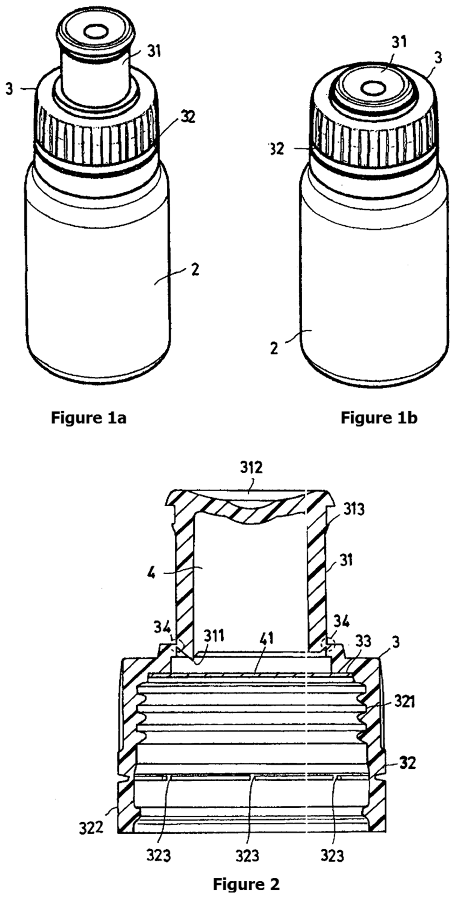

One of the numerous suitable containers of the prior art based on the system "closure storage cap - vial" is described in detail in EP 1 623 932 B1, which is incorporated herein by reference in its entirety. The features and advantages of the "closure storage cap - vial" system, which is used, in a non-limited but indicative and illustrative way in the present invention, are better understood with reference to the accompanying Figures 1 and 2 wherein:
- Figure 1a shows a view of the system "closure storage cap - vial" according to the present invention before the mixing of the ingredients enclosed in the vial 2 and in the closure storage cap 3, respectively, while, Figure 1b shows the system "closure storage cap - vial" according to the present invention after the mixing of the ingredients enclosed in the vial 2 and in the closure storage cap 3, respectively.
- Figure 2 shows a cross-section of the internal configuration of the closure cap 3 which adapted onto vial 2.

With reference to EP 1 623 932 B1 and Figures 1a, 1b and 2, a preferable container used in the present invention consists of the inner threaded closure storage cap 3 which can be screwed onto the vial. In a further embodiment the closure storage cap could be applied onto the vial by applying pressure or in equivalent ways ensuring that the vial will remain hermetically sealed.

In detail, the closure storage cap 3 comprises a capsule, preferably cylindrical and internally hollow, having an upper portion 31 that forms an internal reservoir 4, thus setting up the storage space for the solid mixture comprising tryptophan, 5-hydroxytryptophan and melatonin, which is going to be mixed with the liquid preparation of magnesium pidolate housed in vial 2. The closure storage cap 3 further comprises breaking means at the base of reservoir 4 that can be activated with pressure and manually from the top of said capsule. According to this embodiment, the closure storage cap system, the reservoir and the breaking means are made in a single body from the same material, preferably plastic. The closure storage cap has a lower portion 32, with a greater diameter than the diameter of the upper portion 31, equipped with fixed connective means to the vial 2, such as an internal threading 321. The lower portion 32 further comprises an annular closing seal 322 with the vial 2, of the same type as those present on the lids of drink bottles. In particular, the annular seal 322 is connected with the lower portion 32 through joining elements 323 with predetermined breakability. The base of the reservoir 4 is made through a membrane 41 welded onto the edge 33, aiming to the isolation of reservoir 4 from the vial 2 when the closure storage cap is fitted onto the mouth of vial 2. The joining zone between the upper portion 31 and the lower portion 332 of the capsule determines an annular weakening or pre-fracture sector 34. Such breaking means of the reservoir comprise at least one fracture point 311 made along said annular weakening sector 34. The upper portion 31 also comprises a sealing and clasping ring 313, which does not allow the hollow space of the closure cap (upper portion 31) to disassembly from the closure storage cap 3 and fall into vial 2.

The "closure storage cap-vial" system device of the present embodiment operates in the following way. When the user decides to mix the two separately stored preparations that are contained in the container of this embodiment, it suffices to set pressure with a finger onto cavity 312 existing in the upper section 31 of the container in order to break the annular weakening section 34. Then, the upper portion 31 breaks the membrane 41 and penetrates through fracture point 311 to the lower section of the container, causing the solid mixture comprising tryptophan, 5-hydroxytryptophan and melatonin enclosed in the reservoir 4, to fall by gravity into vial 2, thus in contact with the liquid preparation of magnesium pidolate. Once the mixing between the two preparations has occurred in the vial 2, the closure storage cap can be removed, for example unscrewed, thus allowing the user to consume directly the solution created in vial 2.

The closure storage cap of a container according to the present invention, contains a solid mixture comprising melatonin, tryptophan and 5-hydroxytryptophan. According to the present invention, 5-hydroxytryptophane is in the form of Griffonia seeds dried extract, standardized in >99.0% w/w to 5-hydroxytryptophan.

According to the present invention, the solid mixture enclosed in the closure storage cap of the container further comprises a binder, preferably sorbitol, which aims to facilitate the effective mixing of the solid active ingredients in order to achieve homogenization in the final solid mixture.

According to the present invention, the solid mixture enclosed in the closure storage cap of the container further comprises a lubricant, preferably silicon dioxide, which improves the flowability of the final solid mixture.

In accordance to the present invention, the solid mixture, stored in the closure storage cap of the dietary supplement container, is prepared by mixing the solid active ingredients, namely, melatonin, tryptophan and Griffonia seeds dried extract in a suitable vessel in the presence of a binder and a lubricant for as long as required in order to prepare a homogeneous, easy handling solid powder.

In a preferred embodiment, the amount of melatonin in the solid mixture housed in the closure storage cap of the container of the dietary supplement corresponds to 0.5 % of the total quantity of solid mixture.

In a preferred embodiment, the amount of tryptophan in the solid mixture housed in the closure storage cap of the container of the dietary supplement corresponds to 51.3 % of the total quantity of solid mixture.

In a preferred embodiment, the amount of Griffonia seeds dried extract in the solid mixture housed in the closure storage cap of the container of the dietary supplement corresponds to 25.6 % of the total quantity of solid mixture.

In a preferred embodiment, the amount of the binder in the solid mixture housed in the closure storage cap of the container of the dietary supplement corresponds to 20.5 % of the total quantity of solid mixture.

In a preferred embodiment, the amount of the lubricant in the solid mixture housed in the closure storage cap of the container of the dietary supplement corresponds to 2.1 % of the total quantity of solid mixture.

According to the present invention, the vial of the container is filled with 10 mL liquid preparation of magnesium, which is standardized in 129.95 mg divalent magnesium, in the form of magnesium pidolate. The liquid preparation housed in the vial of the container further comprises purified water as a solvent, sweeteners, preferably sodium saccharine and sodium cyclamate, preservatives, preferably sodium benzoate, flavoring agents, preferably orange flavor, and pH adjusters, preferably citric acid.

According to the present invention, the liquid preparation comprising magnesium, which is stored in the vial of the container of the dietary supplement, is prepared by dissolving the solid active ingredient magnesium pidolate in the solvent followed by the addition of taste and flavor enhancers and preservatives.

In a preferred embodiment, the amount of magnesium pidolate in the liquid preparation stored in the vial of the container of the dietary supplement, corresponds to 15 % w/v of the total amount of the liquid preparation.

In a preferred embodiment, the amount of the solvent in the liquid preparation stored in the vial of the container of the dietary supplement, corresponds to 84.05 % w/v of the total amount of the liquid preparation.

In a preferred embodiment, the amount of sweeteners in the liquid preparation stored in the vial of the container of the dietary supplement, corresponds to 0.55 % w/v of the total amount of the liquid preparation.

In a preferred embodiment, the amount of preservatives in the liquid preparation stored in the vial of the container of the dietary supplement, corresponds to 0.30 % w/v of the total amount of the liquid preparation.

In a preferred embodiment, the amount of flavoring agents in the liquid preparation stored in the vial of the container of the dietary supplement, corresponds to 0.10 % w/v of the total amount of the liquid preparation.

According to the present invention, the closure storage cap containing the solid mixture of melatonin, tryptophan and 5-hydroxytryptophane, has a two-fold role: first, it keeps air tight sealed the solid mixture providing protection from light, water vapor and mixing with the magnesium pidolate solution enclosed in the vial, and second, it keeps the vial with the liquid preparation hermetically sealed thus protecting its content till it is ready to use. The average user achieves the mixing of solid mixture comprising melatonin, tryptophan and 5-hydroxytryptophan with the solution of magnesium pidolate by applying pressure on the upper portion of the closure storage cap, thus driving the solid mixture housed therein to enter the vial, thus after simple shaking, resulting to a single-dose oral solution for immediate use directly form the vial.

The present invention is described in the following non-limited explanatory examples.

### Example 1: Preparation of the solid mixture housed in the closure storage cap

The active ingredients in solid form, namely, melatonin, Griffonia seeds extract, tryptophan, sorbitol and colloidal silica are granulated in order to pass through a sieve of 12 mesh. The granulated solid ingredients placed in a suitable blender where they mixed altogether for a period of 30 to 60 minutes. After mixing completion, homogeneity tests performed and upon approval the final granulated blend is ready to be enclosed in the closure storage caps.

In a preferred embodiment, the solid preparation contained per closure storage cap system of the container of the dietary supplement has the following composition:

| **Ingredients in solid form** | **Quantity (mg) per closure storage cap** |
|---|---|
| Melatonin | 1.0 |
| Tryptophan | 100.0 |
| Griffonia Seeds Extract | 50.0 (corresponds to 49.5 mg 5-hydroxytryptophan) |
| Sorbitol | 40.0 |
| Silica dioxide colloidal anhydrous | 4.0 |

### Example 2: Preparation of the solution contained in the vial.

In a stainless steel reactor, a quantity of purified water, corresponding to 75 % of the total quantity used in the formulation, was placed, followed by the addition of magnesium pidolate in portions under continuous stirring. After magnesium pidolate was fully dissolved, the addition of preservatives and sweeteners (sodium saccharine and sodium cyclamate) followed and the mixture was further stirred for 30 minutes. Subsequently, orange flavor was added and the solution was further stirred for another 20 min. Then, pH was adjusted with citric acid in the range between 3.5 and 5.0. Finally, purified water was added up to the final volume, followed by stirring for another 30 min. The final solution was filtered through membrane filter of 40 µm and then is bottled in the vial which subsequently is sealed with the closure storage cap.

In a preferred embodiment the solution contained in the vial has the following composition:

| **Ingredients in liquid form (final volume 10 mL)** | **Quantity (mg) per vial** |
|---|---|
| Magnesium pidolate | 1500 (corresponds to 129,95 mg Mg²⁺) |
| Sodium Saccharine | 5.0 |
| Sodium Cyclamate | 50.0 |
| Sodium Benzoate | 30.0 |
| Orange flavor | 10.0 |
| Citric acid | qs pH=4.5 |
| Purified Water | qs 10 mL |

According to the present invention, the device of the closure storage cap containing 195 mg of a solid mixture comprising melatonin, tryptophan and 5-hydroxytryptophan, with the vial containing 10 mL of a solution comprising of magnesium pidolate, provides upon its use a single-dose of the dietary supplement, which serves to the normal function of the central nervous system, reduces the time until onset to sleep and improves the effects of biological de-synchronization.

The invention also refers to the following numbered embodiments:
1. Dietary supplement comprising, melatonin, tryptophan, 5-hydroxytryptophan and magnesium pidolate, characterized in that melatonin, tryptophan and 5-hydroxytryptophan altogether stored, in solid form, in the closure cap of a container based on the system "closure storage cap-vial", and magnesium pidolate is stored in liquid form in the vial of said container, furthermore, characterized in that the solid mixture comprising melatonin, tryptophan and 5-hydroxytryptophan and the solution of magnesium pidolate are mixed just before their use to form a single-dose oral solution for direct consumption.
2. Dietary supplement according to item 1, wherein, the solid composition contained in the closure cap is standardized in 0.5 % w/w melatonin, 51.3 % w/w tryptophan and 25.4 % w/w 5-hydroxytryptophan.
3. Dietary supplement according to item 1 and 2, wherein the liquid composition in the vial is standardized in 15 % w/v magnesium pidolate.

### Literature

| | |
|---|---|
| EP 1 623 932 B1 | Closing device with chamber for a container of substances to be kept separate until dispensing. |

## Claims

1. Dietary supplement comprising, melatonin, tryptophan, 5-hydroxytryptophan and magnesium pidolate, **characterized in that** melatonin, tryptophan and 5-hydroxytryptophan altogether stored, in solid form, in the closure cap of a container based on the system "closure storage cap-vial", and magnesium pidolate is stored in liquid form in the vial of said container, furthermore, **characterized in that** the solid mixture comprising melatonin, tryptophan and 5-hydroxytryptophan and the solution of magnesium pidolate are mixed just before their use to form a single-dose oral solution for direct consumption.

2. Dietary supplement according to claim 1, wherein, the solid composition contained in the closure cap is standardized in 0.5 % w/w melatonin, 51.3 % w/w tryptophan and 25.4 % w/w 5-hydroxytryptophan.

3. Dietary supplement according to claim 1 or 2, wherein the liquid composition in the vial is standardized in 15 % w/v magnesium pidolate.

4. Dietary supplement according to any one of the claims 1 to 3, wherein 5-hydroxytryptophane is in the form of Griffonia seeds dried extract, standardized in >99.0% w/w to 5-hydroxytryptophan.

5. Single-dose system device comprising a vial and a closure storage cap,
wherein the closure storage cap encloses a solid mixture comprising melatonin, tryptophan, 5-hydroxytryptophan, a binder, preferably sorbitol, and a lubricant,
wherein the vial contains a liquid preparation, preferably 10 mL, comprising magnesium in the form of magnesium pidolate,
wherein the closure storage cap and the vial are configured such that, upon activation, the solid mixture is brought into contact with the liquid preparation to form a solution just prior to use, and the closure storage cap is removable to allow the user to consume the resulting solution directly from the vial.

6. The single-dose system device according to claim 5, wherein, the solid composition contained in the closure cap is standardized in 0.5 % w/w melatonin, 51.3 % w/w tryptophan and 25.4 % w/w 5-hydroxytryptophan.

7. The single-dose system device according to claim 5 or 6, wherein the liquid composition in the vial is standardized in 15 % w/v magnesium pidolate.

8. The single-dose system device according to any of claims 5 to 7, wherein the closure cap comprises a capsule that forms an internal reservoir.

9. Non-therapeutic use of the dietary supplement or single-dose system device according to any of claims 1-8 for reducing the time until onset of sleep, improving biological de-synchronization and reducing fatigue.
